# EUROPEAN PATENT SPECIFICATION

(11) **EP 2 424 552 B1**
(45) Date of publication and mention of the grant of the patent: **01.03.2017**
(21) Application number: 10770350.6
(22) Date of filing: 29.04.2010
(51) Int. Cl.: A61K 36/074, A61P 25/28, A61P 25/16, A61P 25/00

(54) **GANODERMA LUCIDUM EXTRACTS FOR TREATING PARKINSON'S DISEASE**
GANODERMA LUCIDUM EXTRAKTE ZUR BEHANDLUNG VON PARKINSON
EXTRAITS DE GANODERMA LUCIDUM POUR TRAITER LA MALADIE DE PARKINSON

(30) Priority: 29.04.2009 US 173802 P
(43) Date of publication of application: 07.03.2012
(73) Proprietor: PuraPharm International (H.K.) Limited, Hong Kong (HK)
(72) Inventor: CHAN, Bill, Piu, Beijin 100088 (CN); ZHANG, Ruiping, Hong Kong (CN)
(74) Representative: Engelhard, Markus
(86) International application number: PCT/US2010/033005
(87) International publication number: WO 2010/127143

(56) References cited:
- WO-A1-02/094302
- JP-A- 2008 156 240
- US-A1- 2002 006 444
- US-A1- 2004 029 955
- US-A1- 2005 025 785
- US-A1- 2008 118 583
- US-B2- 7 357 933
- LAI ET AL: "Antagonizing beta-amyloid peptide neurotoxicity of the anti-aging fungus Ganoderma lucidum", BRAIN RESEARCH, ELSEVIER, AMSTERDAM, NL, vol. 1190, 13 November 2007 (2007-11-13), pages 215-224, XP022408988, ISSN: 0006-8993, DOI: 10.1016/J.BRAINRES.2007.10.103
- BAO X-F ET AL: "Structural features of immunologically active polysaccharides from Ganoderma lucidum", PHYTOCHEMISTRY, PERGAMON PRESS, GB, vol. 59, no. 2, 1 January 2002 (2002-01-01), pages 175-181, XP004332847, ISSN: 0031-9422, DOI: 10.1016/S0031-9422(01)00450-2
- FUJITA R ET AL: "Anti-androgenic activities of Ganoderma lucidum", JOURNAL OF ETHNOPHARMACOLOGY, ELSEVIER SCIENTIFIC PUBLISHERS LTD, IE, vol. 102, no. 1, 31 October 2005 (2005-10-31), pages 107-112, XP027757391, ISSN: 0378-8741 [retrieved on 2005-10-31]
- Ruiping Zhang ET AL: "Ganoderma lucidum Protects Dopaminergic Neuron Degeneration through Inhibition of Microglial Activation", Evidence-Based Complementary and Alternative Medicine, 16 July 2009 (2009-07-16), pages 1-9, XP55040712, DOI: 10.1093/ecam/nep075 Retrieved from the Internet: URL:downloads.hindawi.com/journals/ecam/ai p/156810.v1.pdf [retrieved on 2012-10-11]
- Xiu-shu, Wu et al.: "Protective effect of lingzhizongdai against nerve cell damnification", Modern Medicine and Hygiene, 2004, pages 1-3, XP002685099, Retrieved from the Internet: URL:http://www.cnki.net/kcms/detail/detail .aspx?dbcode=cjfq&dbname=cjfq2004&filename =xyws200416002&uid=&p= [retrieved on 2012-10-11]
- LIANG-WEI CHEN ET AL: "Chinese Herbs and Herbal Extracts for Neuroprotection of Dopaminergic Neurons and Potential Therapeutic Treatment of Parkinson's Disease", CNS & NEUROLOGICAL DISORDERS, vol. 6, 1 January 2007 (2007-01-01), pages 273-281, XP55040759, ISSN: 1871-5273
- HONG-BO ZHAO ET AL: "Polysaccharide Extract Isolated From Ganoderma lucidum Protects Rat Cerebral Cortical Neurons From Hypoxia/Reoxygenation Injury", JOURNAL OF PHARMACOLOGICAL SCIENCES, vol. 95, no. 2, 1 January 2004 (2004-01-01), pages 294-298, XP55040788, ISSN: 1347-8613, DOI: 10.1254/jphs.SC0040011
- DATABASE MEDLINE [Online] US NATIONAL LIBRARY OF MEDICINE (NLM), BETHESDA, MD, US; June 2005 (2005-06), ZHU WEI-WEN ET AL: "[Effect of the oil from ganoderma lucidum spores on pathological changes in the substantia nigra and behaviors of MPTP-treated mice].", XP002685100, Database accession no. NLM15958304
- CHAN, PIU: "Abstracts from the 10th International Hong Kong/Springfield Pan-Asian Symposium on Advances in Alzheimer Therapy", NEUROBIOLOGY OF AGING, TARRYTOWN, NY, US, vol. 29, 21 December 2007 (2007-12-21), pages S1-S28, XP022423002, ISSN: 0197-4580
- Xuanwu Hospital: "View of NCT00224263 on 2007_04_17", , 17 April 2007 (2007-04-17), pages 1-2, XP002685101, Retrieved from the Internet: URL:http://clinicaltrials.gov/archive/NCT0 0224263/2007_04_17 [retrieved on 2012-10-11]
- ANONYMOUS: 'Efficacy and Safety of Lingzhi in Patients With Early Parkinson's Disease - No Study Results Posted - ClinicalTrials.gov' INTERNET ARTICLE, [Online] 15 August 2011, pages 1 - 1, XP055105172 Retrieved from the Internet: <URL:http://clinicaltrials.gov/ct2/show/res ults/NCT00224263> [retrieved on 2014-03-03]
- PATERSON ET AL: "Ganoderma - A therapeutic fungal biofactory", PHYTOCHEMISTRY, PERGAMON PRESS, GB, vol. 67, no. 18, 1 September 2006 (2006-09-01), pages 1985-2001, XP028059799, ISSN: 0031-9422, DOI: 10.1016/J.PHYTOCHEM.2006.07.004 [retrieved on 2006-09-01]
- ALTIOK E ET AL: "Isolation of polyphenols from the extracts of olive leaves (Olea europaea L.) by adsorption on silk fibroin", SEPARATION AND PURIFICATION TECHNOLOGY, ELSEVIER SCIENCE, AMSTERDAM, NL, vol. 62, no. 2, 1 September 2008 (2008-09-01), pages 342-348, XP022758139, ISSN: 1383-5866, DOI: 10.1016/J.SEPPUR.2008.01.022 [retrieved on 2008-02-02]

## Description

### BACKGROUND OF INVENTION

Parkinson's disease (PD) is a common neurodegenerative disease, leading to slowed movement, rigidity, rest tremor and disturbances in balance. With the progression of the disease, many patients develop non-motor symptoms, including anxiety, depression, constipation and dementia.

Although there are drugs that alleviate PD symptoms, chronic use of these drugs is not effective in deterring the progression of PD and has been associated with debilitating side effects. It is therefore of great interest to develop neuroprotective therapies aimed at slowing or even halting the degenerative progression.

Unfortunately, the development of effective neuroprotective therapies has been impeded by a limited knowledge of the pathogenesis of degenerative neurological disorders such as PD. The etiology and pathogenesis responsible for the neuronal degeneration in PD remains unknown. Several lines of evidence support the theory that activation of microglia and inflammatory processes are involved in the cascade of events leading to progressive neuronal degeneration (Kreutzberg, G.W., 1996, Trends Neurosci., 19:312-318; Miller, G., 2005, Science, 308:778-781). Numerous activated microglia are present in the vicinity of degenerating neurons in the substantia nigra of patients with PD (McGeer, P. L. et al., 1988, Neurology, 38:1285-1291).

Microglia, the resident innate immune cells of central nervous system, play a major role in the neuroinflammatory process. Microglia can be activated and cause neurotoxicity through two mechanisms (Block, M. L. et al., 2007, Nat. Rev. Neurosci., 8:57-69). First, microglia can initiate neuron damage by recognizing inflammatory triggers, such as LPS and other toxins (Gao, H. M. et al., 2002, J. Neurochem., 81:1285-1297), becoming activated and producing neurotoxic pro-inflammatory factors and cytokines. Consequently, these factors can deplete the antioxidant of DA neurons, impair mitochondrial function, inhibit the re-uptake of glutamate (Persson, M. et al., 2005, Glia, 51:111-120), and initiate CNS tissue damage (Taupin, V. et al., 1997, European Journal of Immunology, 27:905-913). In addition, cytokines such as TNF-α can activate other resting microglia, potentiating inflammatory response that lead to auto-implication of ROS, NO, and superoxide radicals to form highly oxidizing peroxynitrite species (Mosley, R. L. et al., 2006, Clin. Neurosci. Res., 6:261-281; Tansey, M. G. et al., 2007, Exp. Neurol., 208:1-25). TNF-dependent microglia activation in the SN creates an environment of oxidative stress through activation of NADPH oxidase (Mander, P. K. et al., 2006, The Journal of Immunology, 176:1046-1052).

IL-1β has been shown to be involved in the development of CNS inflammation through the disruption of the blood brain barrier which facilitates the infiltration of leukocytes into the CNS (Gao, H. M. et al., 2002, J. Neurochem., 81:1285-1297; Wen, L. L. et al., 2007, Exp. Neurol., 205:270-278). NO is membrane permeable, excessive accumulation NO could react with superoxide to form peroxynitrite which is capable of attacking and modifying proteins, lipids and DNA as well as depleting antioxidant defenses (Persson, M. et al., 2005, Glia, 51:111-120; Taupin, V. et al., 1997, European Journal of Immunology, 27:905-913). Much of the microglial-derived ROS such as superoxide cannot efficiently traverse cellular membranes, making it unlikely that these extracellular ROS gain excess to dopaminergic neurons and trigger intra-neuronal toxic events; however, superoxide can rapidly react with NO in the extracellular space to form a more stable oxidant, which can readily cross cell membranes and damage intracellular components in neighboring neurons (Mosley, R. L. et al., 2006, Clin. Neurosci. Res., 6:261-281).

All these factors can activate a key transcription factor, NF-κB, which can upregulate pro-apoptotic genes leading to neuronal death (Baeuerle, P. A. and Heknel, T., 1994, Annu. Rev. Immunol., 12:141-179; Delhase, M. et al., 2000, Nature (London), 406:367-368).

Second, microglia can become overactivated in response to neuronal damage, which is then toxic to neighbouring neurons (Block, M. L. and Hong, J. S., 2005, Prog. Neurobiol., 76:77-98; Teismann, P. et al., 2003, Mov. Disord., 18), resulting in a perpetuating cycle of neuron death.

Several studies reveal that damaged DA neurons release matrix metalloproteinase 3 (MMP3) (Kim, Y. S. et al., 2005, J. Neurosci., 25:3701-3711), α-synuclein (Zhang, W. et al., 2005, The FASEB Journal, 19:533-542) and neuromelanin (Kim, Y. S. et al., 2005, J. Neurosci., 25:3701-3711; Zecca, L. et al., 2003, Trends Neurosci., 26:578-580) that seem to activate microglia and are implicated in neuronal degeneration in PD. All these events form a vicious circle leading to progressive neuronal degeneration (Fig 9).

Ganoderma lucidum is widely used as an alternative medicine to promote health. Studies have indicated that components extracted from Ganoderma lucidum have pharmacological actions including immunomodulation, suppressing inflammation and scavenging free radicals. In addition, Ganoderma lucidum extracts have been disclosed having anti-tumoral effects (e.g., US Patent Nos. 6,613,754; 7,135,183).

Hong-Bo Zhao et al. Journal of Pharmacological Sciences, 2004, Vol. 95, No. 2, pages 294 - 298 describes a polysaccharide extract isolated from Ganoderma lucidum and its protective effects against hypoxia/reoxygenation injury in red cortical neurons.

Chan, Piu, Neurobiology of Aging, 2007, Vol. 29, pages S1 - S28, XP022423002 discloses an abstract relating to a study exploring the clinical efficacy of Ganoderma lucidum in patients with Parkinson's Disease. No information is provided with respect to the type of extract nor its method of production.

US 7,357,933 relates to methods of treating Parkinson's Disease. It discloses the use of powders obtained from Ganoderma lucidum spores.

However, there have been no previous reports that Ganoderma lucidum could attenuate the inflammatory responses of microglial cells to exogenous or endogenous stimulus and/or protect against degeneration of dopaminergic neurons.

### BRIEF SUMMARY

The subject invention provides Ganoderma lucidum extracts for use in a method for treating Parkinson's Disease (PD) as set forth in the appended claim. All other embodiments are described for illustrative purposes only, and any statement that they would also be part of the invention or other, similar statements, have to be seen in the context of the application as filed and do not alter the scope of the claim.

In accordance with the subject invention, Ganoderma lucidum extracts have been found to be neuroprotective. In a specific embodiment, Ganoderma lucidum extracts can be used according to the subject invention to inhibit the activation of microglia.

In one embodiment, the protective effect of Ganoderma lucidum extracts is attributable to the ability of Ganoderma lucidum to inhibit the production of microglia-derived toxic factors (NO, TNF-α, IL-1β and superoxide) both by LPS and cell membrane exposed to MPP⁺. Thus, Ganoderma lucidum can be used according to the subject invention for the treatment of Parkinson's Disease (PD).

In one aspect, also disclosed is a method of inhibiting the activation of microglia in substantia nigra. In a preferred embodiment, this method comprises administration of an effective amount of Ganoderma lucidum extracts to a subject in need of such treatment.

Advantageously, the side effects of Ganoderma lucidum are minimal, which makes it suitable for long-term use in humans.

Thus, the subject invention provides a Ganoderma lucidum extract for use in a method for the treatment of a patient suffering from Parkinson's Disease (PD) comprising administering to the patient in need an effective amount of a Ganoderma lucidum extract, in accordance with the appended claim.

### BRIEF DESCRIPTION OF THE SEQUENCES

**SEQ ID NO:1** is a forward primer of the interleukin-1 beta (IL-1β) according to the subject invention.
**SEQ ID NO:2** is a reverse primer of the interleukin-1 beta (IL-1β) according to the subject invention.
**SEQ ID NO:3** is a forward primer of the tumor necrosis factor alpha (TNF-α) according to the subject invention.
**SEQ ID NO:4** is a reverse primer of the tumor necrosis factor alpha (TNF-α) according to the subject invention.

### BRIEF DESCRIPTION OF DRAWINGS

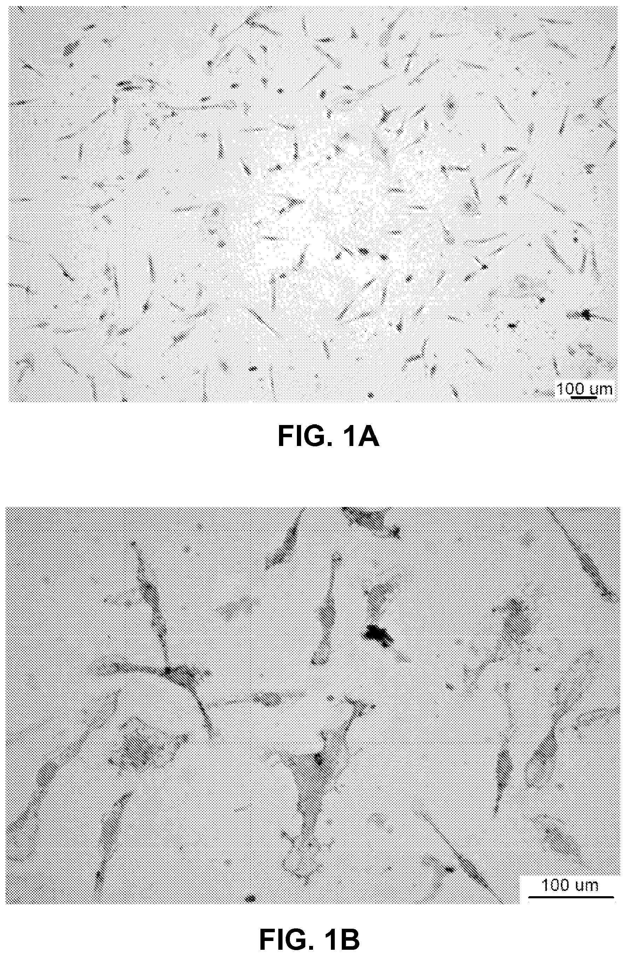

**Figure 1A-D** shows the morphology of rat microglia cells labeled with OX-42. Rat microglia were incubated for 24 hours with vehicle (a, 100×; b, 400×), LPS 0.25 µg/ml, (c, 100×; d, 400×). Note that microglia after being treated with LPS, transformed into an amoeboid morphology. Scale bar represents 100 µm.

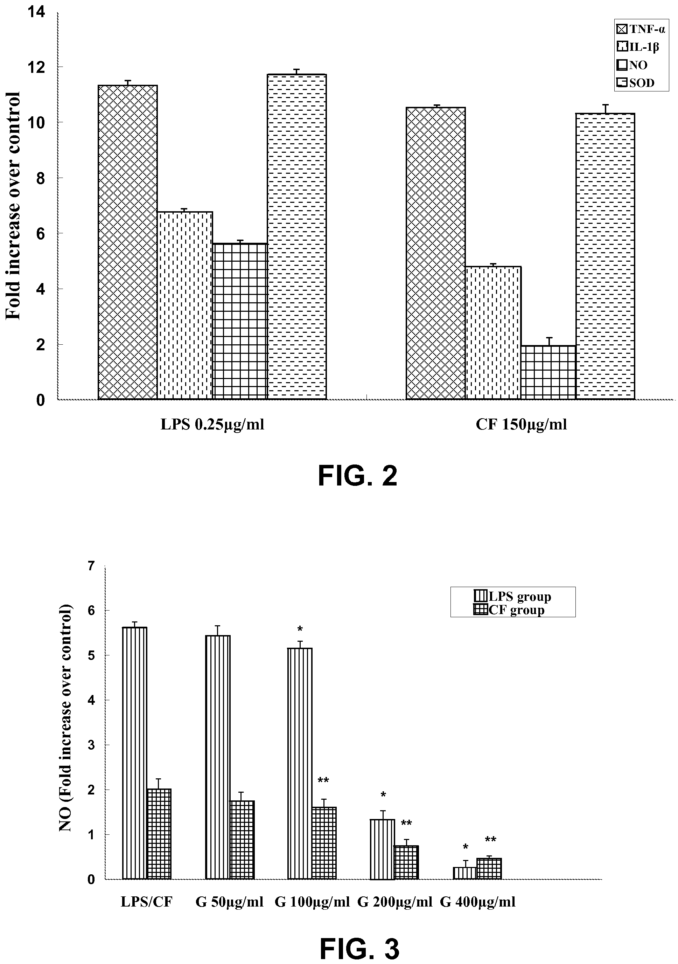

**Figure 2** shows activating effects of LPS and MPP⁺-treated MES 23.5 cell membranes (CF) on microglia. Microglial activation was determined by measuring the levels of TNF-α, IL-1β, NO and superoxide.
**Figure 3** shows the effects of Ganoderma on LPS or CF-stimulated production of nitric oxide (NO). Cultures were treated with vehicle, or indicated concentrations of Ganoderma 30 minutes prior to treatment with 0.25 µg/ml LPS or 150µg/ml CF (control). Culture supernatants were collected and assayed for NO. Data are expressed as fold increase of control group and presented as means ± S.D. of two experiments performed in triplicate: *p < 0.01 compared with LPS-treated cultures, and **p < 0.05 compared with the group of CF.

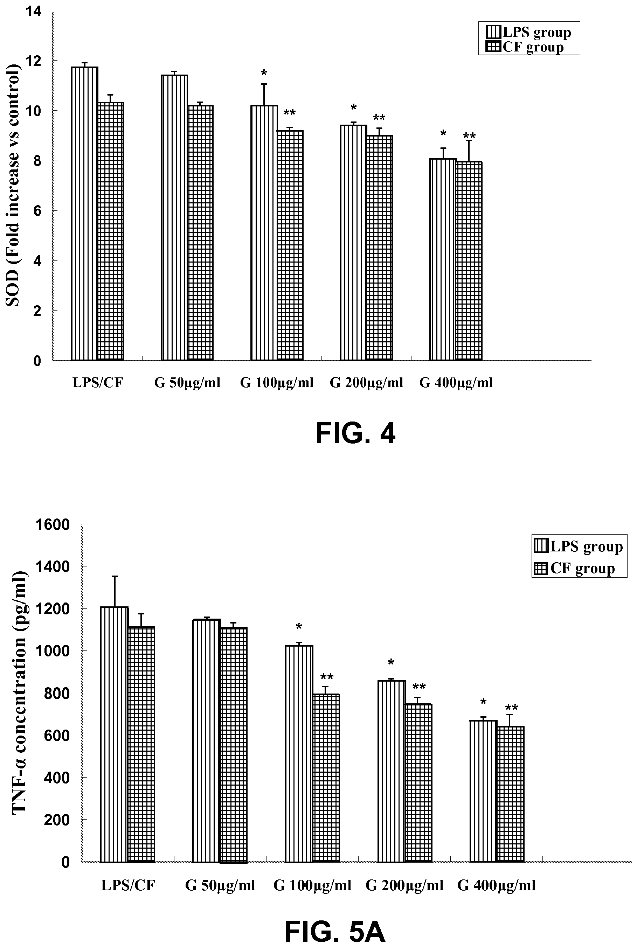

**Figure 4** shows the effects of Ganoderma on LPS or CF-generated production of superoxide. Cultures were treated with vehicle, or indicated concentrations of Ganoderma 30 minutes prior to treatment with 0.25 µg/ml LPS or 150µg/ml CF (control). Superoxide generation was measured with the SOD assay kit-WST. Data are expressed as fold increase of control group. The results are the means ± S.D of triplicate determinations and are representative of two separate experiments: *p < 0.001 compared with LPS-treated control, and **p < 0.05 compared with the group of CF.
**Figure 5A-B** shows the effects of Ganoderma on LPS or CF-induced release of TNF-α and IL-1β. Cultures were treated with vehicle, or indicated concentrations of Ganoderma 30 minutes prior to treatment with 0.25 µg/ml LPS or 150µg/ml CF (control). TNF-α and IL-1β levels were determined as described in Material and Methods. Data represents the means ± S.D. of two experiments performed in duplicate: *p < 0.05, ** p < 0.001 versus control group. #p < 0.001, ## p < 0.001 versus control group.

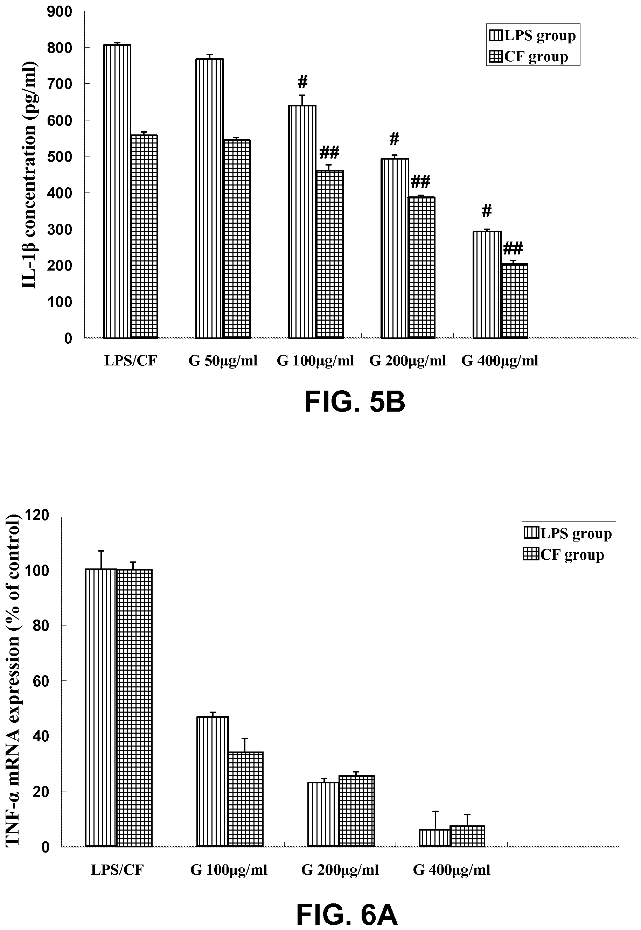

**Figure 6A-B** shows the effects of Ganoderma on mRNA levels of various inflammatory cytokines in microglial cells. Total RNA was extracted and then subjected to real-time PCR. Data are expressed as percentage of the control group (LPS or CF group) calculated from the average threshold cycle values and presented as the mean ± S.D. Determinations were performed in triplicate from the RNA samples of a set of experiments. Independent RNA preparations from different sets of cultures were prepared and used for replicate analysis, which generated similar results.

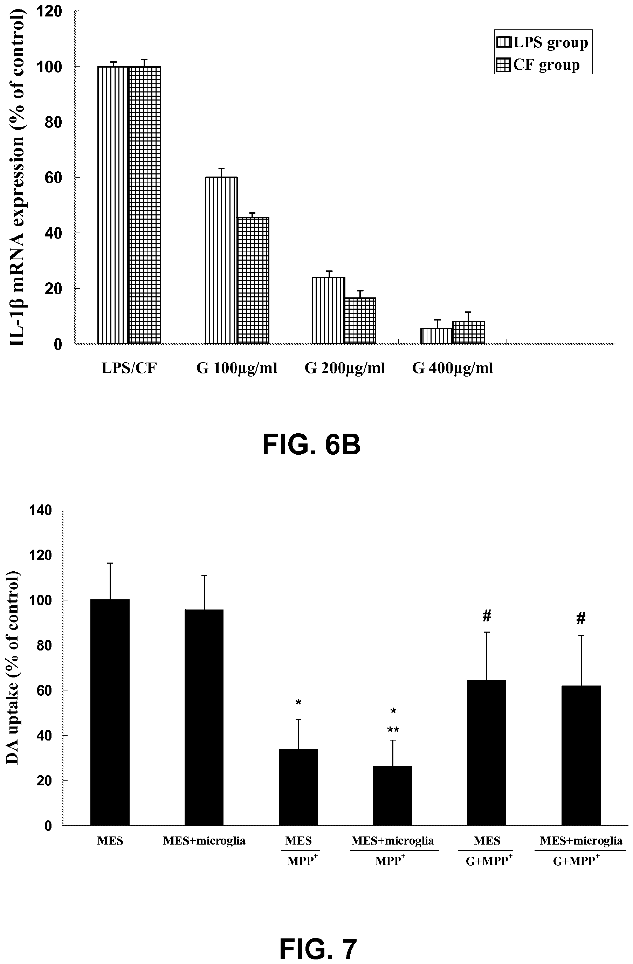

**Figure 7** shows the effect of microglia on the MPP⁺-induced reduction of [³H] dopaminergic uptake in MES 23.5 cell cultures. Uptake of [³H] dopamine was assessed as described in Materials and Methods. The cultures were treated with vehicle (control) and MPP⁺ 100 µm, and the specific groups were pre-treated with Ganoderma 400 µg/ml. The data are expressed as a percent of the dopamine uptake and represented as means ± S.D. Duplicate experiments yielded similar qualitative results: *p < 0.001 compared with control, **p < 0.05 compared with the MPP+-treated MES cultures, #p < 0.001.

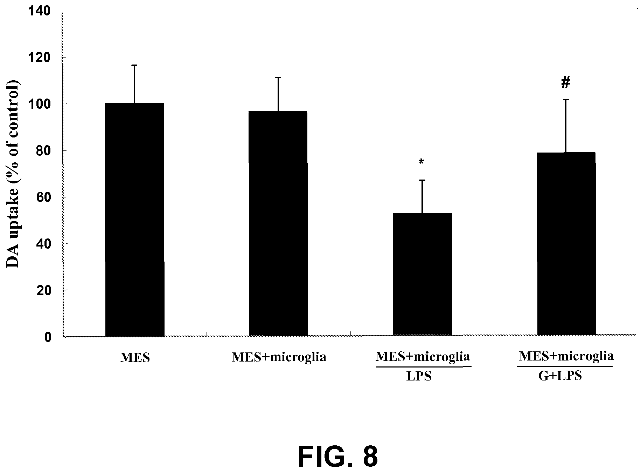

**Figure 8** shows the effect of LPS-activated microglia on the reduction of [³H] dopaminergic uptake in MES 23.5 cell cultures. The cultures were treated with vehicle (control) and LPS 0.25 µg/ml, and the specific group was pre-treated with Ganoderma 400 µg/ml. The data are expressed as a percent of the dopamine uptake and represented as means ± S.D: *p < 0.05 compared with the control group, #p < 0.01 compared with LPS group

### DETAILED DISCLOSURE

The subject invention, provides Ganoderma lucidum extracts for use in a method for treating Parkinson's Disease (PD) in accordance with the appended claim. In accordance with the subject invention, Ganoderma lucidum extracts have been found to be neuroprotective. In a specific embodiment, Ganoderma lucidum extracts can be used according to the subject invention to treat Parkinson's Disease (PD).

In one embodiment, the protective effect of Ganoderma lucidum extracts is attributable to the ability of Ganoderma lucidum to inhibit the production of microglia-derived toxic factors. These factors may be, for example, NO, TNF-α, IL-1β and/or superoxide. Thus, because activated microglia are believed to be a cause of neuronal degeneration, Ganoderma lucidum extracts can be used according to the subject invention for the treatment of degenerative neurological disorders.

In one aspect, also disclosed is a method of inhibiting the activation of microglia in substantia nigra. In a preferred embodiment, this method comprises administration of an effective amount of Ganoderma lucidum extracts to a subject in need of such treatment.

Thus, the subject invention provides Ganoderma lucidum extracts for use in methods for the treatment of a patient suffering from Parkinson's Disease (PD) comprising administering to the patient an effective amount of a Ganoderma lucidum extract.

Advantageously, the side effects of Ganoderma are minimal, which makes it suitable for long-term use in humans.

The Ganoderma lucidum extracts are prepared from the fruiting body of Ganoderma lucidum with methanol by low temperature extraction. In a specific embodiment, the yield of polysaccharide is about 0.6% (w/w) in terms of the fruiting body of Ganoderma lucidum and ergosterol being about 0.35% (w/w).

In accordance with the subject invention, Ganoderma lucidum extracts were found to provide significant inhibition of microglial activation by reducing the production of microglia-derived NO, TNF-α, IL-1β and superoxide (Figures 3, 4, and 5). Ganoderma lucidum, in a concentration-dependent manner, decreased the levels of NO, TNF-α, IL-1β and superoxide induced by activation of microglia. The microglial inhibition offered by Ganoderma lucidum was further confirmed by the mRNA expression of TNF-α and IL-1β, consistent with its ability to reduce TNF-α and IL-1β production.

In addition to inhibiting the microglial activation, Ganoderma lucidum can be used to protect the dopaminergic neurons by blocking the neurodegeneration induced by microglia. In PD, nigral cell degeneration is associated with, or even preceded by, microglial activation that is possibly initiated by environmental or endogenous toxic reactions. Microglial activation (induced by LPS) is capable of initiating neurondegeneration, and microglia could deteriorate the MPP⁺-induced dopaminergic neurodegeneration. Advantageously, with the use of Ganoderma lucidum, the microglial-derived damage is reversed and DA uptake significantly increased (Figures 7 and 8). In the MPP⁺ model, the protective functions of the Ganoderma lucidum were no different on neuron-glia co-cultures or MES 23.5 cell co-cultures.

Thus, also disclosed are methods of inhibiting the activation of microglia by administering an effective amount of a Ganoderma lucidum extract. Preferably, the concentration of Ganoderma lucidum extracts having contact with the microglial cells is over 100 µg/ml, more preferably over 200 µg/ml, and most preferably over 400 µg/ml.

Also disclosed are methods of treating degenerative neurological disorders comprising administering to a patient in need thereof an effective amount of Ganoderma lucidum extract.

The patient that can be treated according to the subject invention herein can be any organism, including mammals, to which treatment with the Ganoderma lucidum extracts are provided. Mammalian species that can benefit from the disclosed compounds and methods of treatment include, but are not limited to, apes, chimpanzees, orangutans, humans, monkeys; and domesticated animals (i.e., pets) such as horses, dogs, cats, mice, rats, guinea pigs, and hamsters. Advantageously, the subject invention can be used long term for protective purposes or for treatment of developed diseases and conditions.

The degenerative disease that can be treated with a Ganoderma lucidum extract in accordance with the subject invention is Parkinson's disease.

Another aspect of the invention provides a composition comprising a Ganoderma lucidum extract. The composition may also include pharmaceutically acceptable carriers, additives, or excipients. The proportions of the Ganoderma lucidum extract and other ingredients are determined by the solubility and chemical nature of the extract, chosen route of administration, and standard medical practice.

The therapeutically effective amount will vary with the condition to be treated, its severity, the treatment regimen to be employed, and the pharmacokinetics of the agent used, as well as the patient to be treated.

The Ganoderma lucidum extracts of the subject invention can be formulated according to known methods for preparing pharmaceutically useful compositions. Formulations are described in a number of sources, which are well known and readily available to those skilled in the art. For example, Remington's Pharmaceutical Science (Martin E W [1995] Easton Pa., Mack Publishing Company, 19th ed.) describes formulations that can be used in connection with the subject invention.

Formulations suitable for parenteral administration include, for example, aqueous sterile injection solutions, which may contain antioxidants, buffers, bacteriostats, and solutes, which render the formulation isotonic with the blood of the intended recipient; and aqueous and nonaqueous sterile suspensions, which may include suspending agents and thickening agents. The formulations may be presented in unit-dose or multi-dose containers, for example sealed ampoules and vials, and may be stored in a freeze dried (lyophilized) condition requiring only a sterile liquid carrier, for example, water, for injections. Extemporaneous injection solutions and suspensions may be prepared from sterile powder, granules, tablets, etc. It should be understood that in addition to the ingredients particularly mentioned above, the formulations of the subject invention can include other agents conventional in the art having regard to the type of formulations in question.

The Ganoderma lucidum extracts of the subject invention can also be formulated consistent with traditional Chinese medicine practices. The composition and dosage of the formulation that are effective in the treatment of a particular disease, condition or disorder will depend on the nature of the disease, condition or disorder by standard clinical techniques.

The traditional Chinese medicine in prescription amounts can be readily made into any form of drug, suitable for administering to humans or animals. Suitable forms include, for example, tinctures, decoctions, and dry extracts. These can be taken orally, applied
through venous injection or mucous membranes. The active ingredient can also be formulated into capsules, powder, pallets, pastille, suppositories, oral solutions, pasteurized gastroenteric suspension injections, small or large amounts of injection, frozen power injections, pasteurized powder injections and the like. All of the above-mentioned methods are known to people skilled in the art, described in books and commonly used by practitioners of herbal medicine.

A tincture is prepared by suspending herbs in a solution of alcohol, such as, for example, wine or liquor. After a period of suspension, the liquid (the alcohol solution) may be administered for example, two or three times a day, one teaspoon each time.

A decoction is a common form of herbal preparation. It is traditionally prepared in a clay pot, but can also be prepared in glass, enamel or stainless steel containers. The formulation can be soaked for a period of time in water and then brought to a boil and simmered until the amount of water is reduced by, for example, half.

An extract is a concentrated preparation of the essential constituents of a medicinal herb. Typically, the essential constituents are extracted from the herbs by suspending the herbs in an appropriate choice of solvent, typically, water, ethanol/water mixture, methanol, butanol, iso-butanol, acetone, hexane, petroleum ether or other organic solvents. The extracting process may be further facilitated by means of maceration, percolation, repercolation, counter-current extraction, turbo-extraction, or by carbon-dioxide hypercritical (temperature/pressure) extraction. After filtration to rid of herb debris, the extracting solution may be further evaporated and thus concentrated to yield a soft extract (extractum spissum) and/or eventually a dried extract, extracum siccum, by means of spray drying, vacuum oven drying, fluid-bed drying or freeze-drying. The soft extract or dried extract may be further dissolved in a suitable liquid to a desired concentration for administering or processed into a form such as pills, capsules, injections, etc.

### Materials and Methods

### Materials

Ganoderma lucidum extracts were generously provided by PuraPharm Corporation (Guangxi, CN). The extracts were prepared from the fruiting body with methanol and low temperature extraction technology. The extracts used were defined by a content of polysaccharides and ergosterin. The yield of polysaccharide was 0.6% (w/w) in terms of the fruiting body of Ganoderma, and ergosterol was 0.35%. Ganoderma lucidum was resolved in phosphate-buffered saline. Cell culture reagents were obtained from Gibco (Grand Island, NY) and [3H] dopamine (DA) was purchased from PerkinElmer Life Science (Boston, MA). Lipopolysaccharides and Griess reagent were purchased from Sigma (St. Louis, MO). The monoclonal antibody against rat CD11b (OX-42) was obtained from Serotec (Oxford, UK). Diaclone (Besancon, FRA) supplied Rat TNF-α detection ELISA kits, while superoxide Assay Kit-WST and rat IL-1β ELISA kits were obtained from Dojindo, Kyushu, JP and IBL (Gunma, JP), respectively. The real-time PCR reagents were provided by Takara (Tokyo, JP). Cultures of Microglia and MES 23.5 cells

Microglia were isolated and purified from brains of 12-24 hours old Wistar rats supplied by Laboratory Animal Center (Le, W. D. et al., 2001, J. Neurosci., 21:8447-8455). Briefly, after brains were dissected and the meninges removed, the tissues were minced and digested with trypsin (0.25% trypsin-EDTA in 0.1M phosphate buffer) for 20 minutes at 37 °C, triturated with a fire-polished Pasteur pipette and filtered through a 200 µM nylon cell strainer. After centrifugation for 5 minutes at 800rpm, the tissues were suspended into DMEM containing 10% fetal bovine serum (FBS), and seeded in 75cm² flasks at a density of 5×10⁵/ml cells per flask. Two weeks after the seeding, the flasks were shaken at 180rpm for 4 hours, and the floating cells were collected and centrifuged for 5 minutes at 800rpm. The cells were resuspended and plated to 96-well plates for further experimental treatment.

The dopaminergic cell line MES 23.5 was a gift from professor Wei-dong Le, Department of Neurology, Baylor College of Medicine, Houston. The MES 23.5 cells were derived from somatic cell fusion of rat embryonic mesencephalic cells with murine N18TG2 neuroblastoma cells (Crawford, G. D. et al., 1992, J. Neurosci., 12:3392-3398). MES 23.5 cells display many properties of developing neurons of the SN zona compacta and offer several advantages for such initial studies, including greater homogeneity than primary cultures and susceptibility to both free-radical-mediated cytotoxicity and calcium-dependent cell death. MES 23.5 cells were seeded on polylysine-precoated 24-well plates at a density of 104 cells/cm² and maintained in DMEM with Sato's components at 37 °C in a 95% air/5% CO₂ humidified atmosphere incubator. Some of the cultured MES 23.5 cells were co-cultured with microglia.

To study the interaction of reactive microglia with MES 23.5 cells, microglia and MES 23.5 cells were co-cultured in 24-well culture plates. Briefly, the purified microglia were plated at a density of 1 x 10⁴/well 1 day before addition of MES 23.5 cells at a ratio of 2:1 (MES 23.5 to microglia). The co-cultures were maintained in Sato's conditioned medium containing 2% heat-inactivated fetal bovine serum. The cultures of microglia or MES 23.5 cells alone or together were treated for 24 hours with lipopolysaccharide (LPS, 0.25µg/ml) as a positive control, Ganoderma lucidum extracts (50-400 µg/ml) or MES 23.5 cell membrane constituents (150 µg/ml) (Le, W. D. et al., 2001, J. Neurosci., 21:8447-8455).

### Immunocytochemistry

Paraformaldehyde-fixed cell cultures were immunostained as described previously (Gao, H. M. et al., 2002, J. Neurosci., 22:782-790). Microglia was stained with a monoclonal antibody OX-42. Briefly, cell cultures were treated for 15 minutes with 3% H₂O₂, then blocked with appropriate normal serum followed by incubation overnight at 4 °C with a primary antibody diluted in antibody diluents (Gao, H. M. et al., 2002, J. Neurosci., 22:782-790). After incubation with an appropriate biotinylated secondary antibody and then the ABC reagents, the bound complex was visualized by color development with 3,3'-diaminobenzidine (DAB). Images were recorded with a Nikon inverted microscope.

### Preparation of MES 23.5 Cell Membrane Fraction

After exposure to MPP+ 10µM for 24 h, the MES 23.5 cells were harvested in a buffer containing 0.25 M sucrose, 100 mM PBS, 1 mM MgCl₂, 1 mM EDTA, and 2 µM protease inhibitor PMSF, and homogenized with a glass-teflon homogenizer (Le, W. D. et al., 2001, J. Neurosci., 21:8447-8455). Then the homogenate was centrifuged at 8000×g for 10 min at 4°C to remove the crude nuclear fractions. The supernatants were again centrifuged at 100,000 × g for 60 minutes at 4°C. The precipitates were homogenized and suspended in culture medium and used as the neuronal membrane fractions.

### High-Affinity [3H] Dopamine Uptake Assay

Cells in each well were washed with 1 ml of Krebs-Ringer buffer (16 mM NaH₂PO₄, 16 mM Na₂HPO₄, 119 mM NaCl, 4.7 mM KCl, 1.8 mM CaCl₂, 1.2 mM MgSO₄, 1.3 mM EDTA, and 5.6 mM glucose; pH 7.4). The cells were then incubated with 10 nM [3H]dopamine in Krebs-Ringer buffer (10 µl/well) for 30 min at 37 °C (Gao, H. M. et al., 2002, J. Neurosci., 22:782-790). Nonspecific uptake of dopaminergic was determined in parallel wells receiving both dopaminergic and 1 mM nomifensine (10 µl/well), an inhibitor of neuronal high-affinity dopamine uptake. Afterward, the cells were washed three times with ice-cold Krebs-Ringer buffer (1 ml/ well) and lysed with 1 N NaOH (0.5 ml/well). After mixing the lysate with 3 ml of scintillation fluid overnight, radioactivity was determined with Perkin Elmer 1450LSC Luminescence Counter (Waltham, USA.). Specific uptake was determined by subtracting the nonspecific counts for the total activity.

### NO Assay

The production of NO was quantified by measuring the released NO metabolites (nitrates and nitrites) with Griess reagent (Mayer, A. M., 1998, Medicina (B Aires), 58:377-385). After a 24 hours exposure to LPS/cell fraction, the culture medium samples were collected and prepared cell-free by centrifugation. The medium was incubated with the same volume of Griess reagent at room temperature for 10 minutes before measuring absorbance at 540nm in a LP-400 ELISA reader (Diagnostics Pasteur, Marne-la-Coquette, France) with appropriate standards.

### TNF-α, IL-1β and Superoxide assay

Samples were prepared similar to NO samples and the production of these factors were determined using rat TNF-α kit, rat IL-1β ELISA kit and superoxide Assay Kit-WST according to the manufacturer's instructions. Measurements were conducted at 450 nm.

### RNA Isolation and Real-time PCR

Total RNA was extracted from primary microglial cells using RNAprep Kit according to the manufacturer's specifications. RNA was primed with random 9 mers and converted into cDNA by reverse transcription (RT) using AMV reverse transcriptase by following the
manufacturer's recommended protocol (Schell, J. B. et al., 2007, J. Neuroimmunol., 189:75-87; Liu, B. et al., 2000, J. Pharmacol. Exp. Ther., 293:607-617). The resulting cDNA was then subjected to real-time PCR with SYBR Premix Ex Taq containing a final concentration of 1 × SYBR Green (Molecular Probes) and 0.2 µM of the primer set of interest in a 20 µl reaction. The PCR mixture was run in the DNA engine Opticon 2 (MJ research; Waltham, MA). After an initial 10-second 95 °C denaturation step, the reaction was run through 35 cycles at 95 °C for 5 s, 60 °C for 30 s, and 80 °C for 1 s. Melting curve analysis was executed to ensure the resulting products from the reaction had equivalent and appropriate melting temperatures. The specific primers used are listed in Table 1 (Schell, J. B. et al., 2007, J. Neuroimmunol., 189:75-87). The quantification of target transcripts was based on a calibration curve. The "housekeeping" gene β-actin was targeted for an internal control gene. The test gene data were normalized by corresponding β-actin data.

**Table 1. Primers and condition for amplification of IL-1β and TNF-α**

| Sequence name | Abbreviation | Accession # | Forward primer | Reverse primer | Product size |
|---|---|---|---|---|---|
| Interleukin-1 beta | IL-1β | NM_008361 | CCGTGGACCTT CCAGGATGA (SEQ ID NO:1) | GGGAACGTCAC ACACCAGCA (SEQ ID NO:2) | 102 bp |
| Tumor necrosis factor alpha | TNF-α | NM_013693 | CCACCACGCTC TTCTGTCTA (SEQ ID NO:3) | AGGGTCTGGGC CATAGAACT (SEQ ID NO:4) | 116 bp |

### Statistical Analysis

Data were expressed as the means ± S.D. Statistical significance was assessed with an analysis of variance (ANOVA) followed by LSD post hoc test using SPSS 11.5. A value of p < 0.05 was considered to be statistically significant.

### EXAMPLE 1 - MICROGLIAL ACTIVATION INDUCED BY LPS AND MPP+- TREATED DOPAMINERGIC CELL MEMBRANES

To establish models of microglia activation in neurodegeneration, LPS and MPP+- treated dopaminergic cell membranes were used as stimuli in either microglia culture or dopaminergic neuron (MES23.5 cell line) and microglia co-cultures.

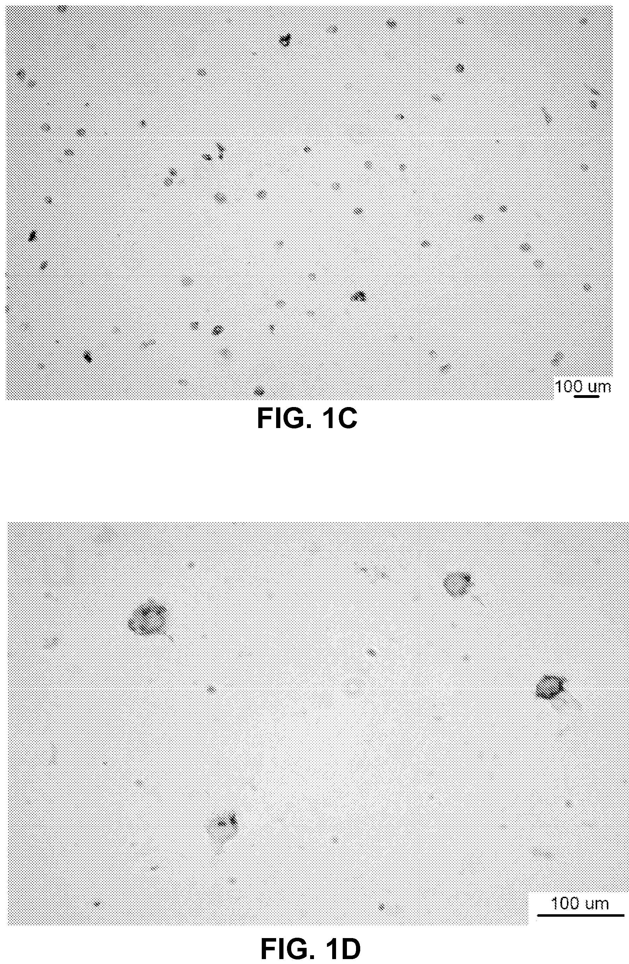

Microglia, were visualized by staining for the CR3 complement receptor using monoclonal antibody OX-42. The purity of microglia cultures is ∼ 95%. The quiescent microglia displayed either ramified shapes or bipolar or multipolar processes (Figure 1 a and b). The activated microglia displayed amoeboid morphology (Figure 1c and d).

Of the numerous neurotoxic factors, NO, TNF-α, IL-1β and superoxide may be major mediators of dopaminergic neurodegeneration elicited by microglial activation. The LPS-induced microglial activation was characterized by measuring the levels of TNF-α and IL-1β, two well-documented cytokines reflecting microglial activation, and the levels of several reactive oxygen species (ROS, NO and superoxide) released from activated microglia.

Unstimulated microglia produces very low amounts of any cytokine. After being exposed to LPS (0.25 µg/ml), the levels of TNF-α and IL-1β were increased by 6-11 fold, and the levels of NO and superoxide were elevated up to 5-11 fold in the microglia culture medium (Figure 2).

Because MES 23.5 cells activated microglia only after MPP⁺ treatment, the activation effects of MES 23.5 cells membrane fractions (CF) treated with MPP⁺ were examined. After incubation with MPP⁺-treated cell membrane fraction (150 µg/ml), TNF-α and IL-1β production was significantly increased by 4-10 fold (Figure 2). The levels of NO and superoxide from the MPP+ membrane fraction-treated microglial culture medium were also measured and it was found that they were increased by 2-10 fold (Figure 2).

Crude membrane without MPP⁺ or treated with Ganoderma lucidum only had minimal activating effects compared with MPP⁺ membrane fraction.

### EXAMPLE 2 - GANODERMA LUCIDUM PREVENTS THE PRODUCTION OF PRO-INFLAMMATORY FACTORS AND ROS DERIVED FROM MICROGLIA

Microglia can produce cytokines as a consequence of activation (20-22). To elucidate the underlying mechanism of the neuroprotective activity of Ganoderma lucidum, the effect of Ganoderma lucidum on the levels of microglia-derived inflammatory cytokines and ROS were investigated. Microglial cell cultures were pretreated with different dosages (50∼400 µg/ml) of Ganoderma lucidum for 30 minutes followed by exposure to LPS or CF treated with MPP⁺

As shown in Figures 3 and 4, a low dose (50 µg/ml) of Ganoderma lucidum had minimal inhibiting effects, while pretreatment with a higher dose of Ganoderma (100-400µg/ml) potently reduced the increase of NO and SOD caused by LPS or CF in a concentration-dependent fashion.

At the equivalent concentration, Ganoderma lucidum also significantly decreased the release of TNF-α and IL-1β after LPS and CF treated with MPP⁺ (Fig. 5).

### EXAMPLE 3 - GANODERMA LUCIDUM PROTECTS AGAINST MPP⁺-INDUCED DOPAMINERGIC NEURODEGENERATION IN THE PRESENCE AND ABSENCE OF MICROGLIA

To assess inflammation mediated neurotoxicy, dopaminergic MES23.5 neurons were exposed to 100 µM MPP⁺ or 0.25 µg/ml LPS in the absence or presence of microglia co-culture for 24 hr, and neurotoxicity was assessed using [3H] DA uptake assay.

Exposure to MPP⁺ lead to a significant decrease in [3H] DA uptake by about 66% for MES23.5 neurons alone, while an about 74% decrease was noted for MES23.5 and microglia co-cultures (Figure 6).

Pretreatment with 400 µg/ml Ganoderma lucidum significantly protected MPP⁺- induced reduction of [3H] DA uptake, which only decreased by about 35% and 38%, respectively, in the absence and presence of microglia co-cultures.

### EXAMPLE 4 - GANODERMA LUCIDUM PROTECTS AGAINST LPS-INDUCED DOPAMINERGIC DEGENERATION IN THE PRESENCE OF MICROGLIA

When neuron-microglia co-cultures were exposed to 0.25 µg/ml LPS for 24 hr, [3H] DA uptake was significantly reduced by approximately 50% as compared to co-cultures (Figure 7). Pretreatment of co-cultures with 400 µg/ml Ganoderma lucidum also significantly attenuated LPS-induced decrease in [3H] DA uptake (22% loss with Ganoderma lucidum vs 50% loss without Ganoderma lucidum).

### EXAMPLE 5 - GANODERMA LUCIDUM INHIBITS THE INCREASED EXPRESSION OF TNF-A AND IL-1B MRNA BY LPS AND MPP⁺-TREATED MEMBRANE

Synthesis of proinflammatory factors is controlled at several levels. Whereas post-transcriptional, translational, and post-translational mechanisms play important roles, gene transcription appears to be the primary regulatory site. The levels of TNF-α and IL-1β mRNA expression were barely detectable in control cells but were significantly increased by LPS and CF.

Pretreatment of 100∼400 µg/ml Ganoderma lucidum inhibited their expression in a dose-dependent manner. The higher dose of 400µg/ml Ganoderma lucidum provided a 90% protection (Figure 8).

## Claims

1. A Ganoderma lucidum extract for use in a method for treating Parkinson's disease, said method comprising administering, to a subject, preferably a human, in need of such treatment, an effective amount of a Ganoderma lucidum extract,
wherein said Ganoderma lucidum extract is obtained from the fruiting body of Ganoderma lucidum with methanol by low temperature extraction.

## Patentansprüche

1. Extrakt von Ganoderma lucidum zur Verwendung in einem Verfahren zum Behandeln von Parkinsonismus, wobei das Verfahren umfasst: Verabreichen einer wirksamen Menge eines Extrakts von Ganoderma lucidum an einen für eine solche Behandlung bedürftigen Patienten, bevorzugt einen Menschen, wobei der Extrakt von Ganoderma lucidum von dem Fruchtkörper von Ganoderma lucidum mit Methanol mittels Niedrigtemperaturextraktion erhalten wird.

## Revendications

1. Extrait de Ganoderma lucidum pour une utilisation dans un procédé pour traiter la maladie de Parkinson, ledit procédé comprenant l'administration à un sujet, de préférence un être humain qui a besoin de ce traitement, d'une quantité efficace d'un extrait de Ganoderma lucidum,
dans lequel ledit extrait de Ganoderma lucidum est obtenu à partir du carpophore de Ganoderma lucidum avec du méthanol par une extraction à basse température.


### SEQUENCE LISTING

<110> PuraPharm International (HK) Limited Chan, Bill Piu Zhang, Ruiping
<120> NEUROPROTECTIVE GANODERMA COMPOSITIONS AND METHODS OF USE
<130> PURA.102X
<150> 61/173,802
   <151> 2009-04-29
<160> 4
<170> PatentIn version 3.5
<210> 1
   <211> 20
   <212> DNA
   <213> Mus musculus
<400> 1
   ccgtggacct tccaggatga 20
<210> 2
   <211> 20
   <212> DNA
   <213> Mus musculus
<400> 2
   gggaacgtca cacaccagca 20
<210> 3
   <211> 20
   <212> DNA
   <213> Mus musculus
<400> 3
   ccaccacgct cttctgtcta 20
<210> 4
   <211> 20
   <212> DNA
   <213> Mus musculus
<400> 4
   agggtctggg ccatagaact 20